# EUROPEAN PATENT SPECIFICATION

(11) **EP 1 943 359 B1**
(45) Date of publication and mention of the grant of the patent: **29.06.2011**
(21) Application number: 06815683.5
(22) Date of filing: 29.09.2006
(51) Int. Cl.: C12Q 1/68, G01N 33/567, A61K 39/395

(54) **COMPOSITIONS AND METHODS FOR IL13 BIOMARKERS**
ZUSAMMENSETZUNGEN UND VERFAHREN FÜR IL13-BIOMARKER
COMPOSITIONS ET METHODES POUR DES BIOMARQUEURS POUR IL-13

(30) Priority: 30.09.2005 US 722296 P
(43) Date of publication of application: 16.07.2008
(73) Proprietor: Centocor Ortho Biotech Inc., Horsham, PA 19044 (US)
(72) Inventor: SYED, Farhat, Audubon, Pennsylvania 19403 (US); HUANG, Chong, C., Paoli, Pennsylvania 19301 (US); LI, Katherine, Wallingford, Pennsylvania 19086 (US); LIU, Guihua, Chadds Ford, Pennsylvania 19317 (US); LI, Li, Downingtown, Pennsylvania 19335 (US); SHANG, Xiaozhou, West Chester, Pennsylvania 19382 (US)
(74) Representative: Marshall, Cameron John
(86) International application number: PCT/US2006/037856
(87) International publication number: WO 2007/041219

(56) References cited:
- EP-A- 1 347 051
- EP-A- 1 394 274
- US-A- 5 853 697
- US-A1- 2005 036 951
- US-A1- 2005 036 951
- YANG GAOYUN ET AL: "Anti-IL-13 monoclonal antibody inhibits airway hyperresponsiveness, inflammation and airway remodeling." CYTOKINE 21 DEC 2004, vol. 28, no. 6, 21 December 2004 (2004-12-21), pages 224-232, XP002542095 ISSN: 1043-4666
- WALTER D M ET AL: "Critical role for IL-13 in the development of allergen-induced airway hyperreactivity." JOURNAL OF IMMUNOLOGY (BALTIMORE, MD. : 1950) 15 OCT 2001, vol. 167, no. 8, 15 October 2001 (2001-10-15), pages 4668-4675, XP002542096 ISSN: 0022-1767
- YUYAMA NORIKO ET AL: "Analysis of novel disease-related genes in bronchial asthma." CYTOKINE 21 SEP 2002, vol. 19, no. 6, 21 September 2002 (2002-09-21), pages 287-296, XP002542097 ISSN: 1043-4666
- LEE J H ET AL: "Interleukin-13 induces dramatically different transcriptional programs in three human airway cell types." AMERICAN JOURNAL OF RESPIRATORY CELL AND MOLECULAR BIOLOGY OCT 2001, vol. 25, no. 4, October 2001 (2001-10), pages 474-485, XP002542098 ISSN: 1044-1549
- SYED F ET AL: "Identification of interleukin-13 related biomarkers using peripheral blood mononuclear cells" BIOMARKERS, TAYLOR AND FRANCIS, LONDON, GB, vol. 12, no. 4, 1 August 2007 (2007-08-01), pages 414-423, XP008110155 ISSN: 1354-750X
- WILLS-KARP M ET AL: "INTERLEUKIN-13: CENTRAL MEDIATOR OF ALLERGIC ASTHMA" SCIENCE, AMERICAN ASSOCIATION FOR THE ADVANCEMENT OF SCIENCE, US, WASHINGTON, DC, vol. 282, 18 December 1998 (1998-12-18), pages 2258-2260, XP002948381 ISSN: 0036-8075

## Description

### Field of the Invention

The invention relates to an assay method for monitoring the biological response to IL-13 or IL-13 therapy using discovered biomarkers for IL-13.

### Background and Related Art

Asthma is a complex, chronic disorder, with a genetic and an environmental component. It is characterized by reversible airway obstruction, airway hyper-responsiveness, airway inflammation and remodeling. Asthma affects an estimated 15 million Americans and the morbidity and mortality associated with it is on the rise in industrialized countries. Recent reports using murine models of allergic asthma have shown that the Th2 type cytokine IL-13 may play a critical role in the pathogenesis of asthma, either by regulating airway inflammation, mucus hypersecretion or airway hyper-responsiveness thus making it an attractive target for therapeutic intervention.

The role of biomarkers is becoming increasingly important in the clinical development of therapeutics. A biomarker can be an indicator of normal biological processes, disease processes, or pharmacological responses to therapeutic intervention. Their role ranges from, stratifying the patient population in helping to identify responders versus non-responders, to determining the efficacy of the therapeutic. Biomarkers can be a valuable tool in making better decisions that will reduce the cost for drug development and enable therapies to reach the right patient population faster.

The asthmatic population is very heterogeneous in terms of symptoms and response to therapy. Biomarkers can prove very useful in this by identifying responders versus non-responders. Thus leading to the development of more effective therapeutics.

Yang et al., 2004, Anti-IL-13 monoclonal antibody inhibits airway hyperresponsiveness, inflammation and airway remodelling. Cytokine 28: 224-232 discloses a study where an anti-IL-13 monoclonal antibody is applied and evaluated in a chronic mouse model of asthma.

Walter et al., 2001, Critical role for IL-13 in the development of allergen-induced airway hyperreactivity. Journal of immunology 167: 4668-4675 discloses a study on IL-13 deficient mice.

US2005/036951 discloses antibodies and IL-13 as therapeutic agents, but does not disclose anti-IL-13 antibodies.

EP1394274 discloses a method for testing for bronchial asthma or chronic obstructive pulmonary disease by determining expression levels of marker genes that increase when respiratory epithelial cells are stimulated with IL-13, but does not disclose the use of MNT5A or MPIF-1 (CCL23) as markers.

### SUMMARY OF THE INVENTION

This invention discloses the discovery of a panel of genes of which one or more can serve as biomarkers in an anti-interleukin(IL)-13 clinical trial.

The observation originated from data analysis of microarray gene expression profiling of peripheral blood mononuclear cells (PBMCs) from healthy donors. When comparing untreated PBMCs to PBMCs cultured with IL-13 or IL-4 this panel of genes was up regulated in all donors. The observation was confirmed by an independent method -Real Time Polymerase Chain Reaction (PCR) at messenger RNA level and the expression of all these 10 genes was up regulated by IL-13 and IL-4. A subsequent study looking at level of thymus and activation regulated chemokine (TARC) and macrophage derived chemokine (MDC) in the culture media supernatant of PBMCs showed that the neutralization of IL-13 by a fully human anti-interleukin 13 therapeutic antibody, as disclosed in WO 2006/12445 inhibited the IL-13 induced TARC and MDC proteins as detected by ELISA. This disclosure presents evidence that this panel of genes can be used as biomarkers in an anti-IL-13 clinical study for inflammatory disorders such as asthma, emphysema and chronic obstructive pulmonary disease (COPD), and may also play a role in other lung disorders with an inflammatory component.

The panel of genes responsive to IL-13 disclosed provides methods for diagnosis or prognosis and tracking the efficacy of an anti-IL-13 therapy in IL-13 related disorders, including but not limited to, emphysema, asthma and chronic obstructive pulmonary disorder (COPD). Diagnostic and prognostic methods based on detecting these IL-13 responsive genes, or protein, in a sample is also disclosed.

Thus, in accordance with the invention, there is provided a method of monitoring the response to anti-IL13 therapy in a patient undergoing such therapy by using one or more IL-13 biomarkers, which comprises:
(a) Determining the level of expression of at least one of WNT5A or MPIF-1(CCL23) in a tissue sample of a patient;
(b) Measuring the level of at least one of WNT5A or MPIF-1 (CCL23) expression in said tissue of the patient and determining whether the anti-IL-13 therapy is effective in reducing the level of IL-13 or IL-13 related physiological effects.

The method can also include at least one of the biomarkers CCL17 (TARC), CCL22 (MDC), CCL23 (MPIF-1) and CCL26 (Eotaxin 3), CD1A, CD1B, CD1C, IgE CD23A, IL-17R beta.

### FIGURE DESCRIPTIONS

**Figure 1****.** Microarray data showing the average normalized intensity levels of CCL17, CCL22, CCL23 and CCL26 upon treatment of PBMCs from ten donors with IL-13, IL-4, anti-IL-13 mAb or isotype control mAb for 24 hrs.
**Figure 2****.** Real Time PCR (Taqman®) analysis showing the level of A) CCL17 B) CCL22 C) CCL23 D) CCL26 upon treatment of PBMCs from ten donors with IL-13, IL-4, anti-IL-13 mAb or isotype control mAb for 24 hrs. The quantity of each gene is normalized to GAPDH.
**Figure 3****.** Effect of neutralizing IL-13. PBMCs were incubated with IL-13, IL-13 + anti-IL-13 mAb, or IL-13 + isotype control mAb for 24 hr and 48 hr timepoints. The level of A) CCL17 B) MDC C) CCL26 was assessed in the cell culture supernatants using ELISA.

### DETAILED DESCRIPTION

The present invention thus provides a new approach to monitoring anti-IL13 therapy by detecting one or more biomarkers of IL-13, and IL-13 bioactivity.

In summary, we have identified a panel of potential biomarkers, associated with IL-13 biology, which can be useful in an anti-IL-13 clinical trial or for monitoring therapy using methods well known in the art are as disclosed herein. Such a panel of biomarkers can be used as a tool to monitor the efficacy of an anti-IL-13 therapeutic such as an IL-13 antagonist such as a small molecule or a biologic, and provide valuable information in terms of dosing amount and frequency.

The relevance of these genes to disease is very important for them to be effective biomarkers. To that end a number of the genes discussed above have been linked to diseases where IL-13 is believed to play a role. Increased level of TARC has been reported in serum and induced sputum of asthmatics [11], plasma of children during asthma exacerbation [12] and serum of patients with atopic dermatitis [13]. This provides evidence that TARC might be involved in the pathogenesis of such disorders. Eotaxin 3 gene expression has been shown to be upregulated in bronchial biopsies of asthmatics after allergen challenge implying that eotaxin 3 may be important in late-phase eosinophil recruitment to the airways of asthmatics [14]. Also, CCL22 was shown to be up regulated in bronchoalveolar lavage (BAL) after a segmental allergen challenge of asthmatics [15].

Of the panel of potential biomarkers described above, WNT5A and MPIF-1 (CCL23) have not been previously reported to be regulated by IL-13. These are novel IL-13 related biomarkers, which can be useful in any disease where IL-13 plays a major role.

In one embodiment, the improvement is to use of at least one of WNT5A and MPIF-1 (CCL23) as a biomarker for IL-13 antagonist treatment or therapy.

Several methods have been reported for the measurement and any of these methods may be employed in the invention. These include (i) radioimmunoassays and single radial immunodiffusion procedures (Chambers, R. E. and Whicher, J. T. (1983); J. Immunol. Methods 59, 95; Marhaug, G. (1983) supra; Taktak, Y. S. and Lee, M. A. (1991); J. Immunol. Methods 136, 11); (ii) ELISA based assays (Zuckerman, S. H. and Suprenant, Y. M.(1986); J. Immunol. Methods 92, 3743; Dubois, D. Y. and Malmendier, C. L. (1988); J. Immunol. Methods 112, 71-75; Sipe, J. D. et al. (1989); J. Immunol. Methods 125, 125-135; Yamada, T. et al. (1989); Clin. Chim. Acta 179, 169-176; Tino-Casl, M. and Grubb, A. (1993); Arm. Clin. Biochem 30, 278-286); (iii) nephelometric methods (Vermeer, H. et al. (1990); Clin. Chem 36, 1192; Yamada, T. et al. (1993); Ann. Clin. Biochem. 30, 72-76); (iv) an electrophoretic procedure (Godenir, N. L. et al. (1985); J. Immunol. Methods 83, 217); (v) an immunochemiluminescence procedure (Hachem, H. et al. (1991); Clin. Biochem 24, 143-147); (vi) an automated method based on a monoclonal-polyclonal antibody solid phase enzymeimmunoassay (Wilkins, J. W. et al. (1994); Clin. Chem 40(7), 1284-1290); and (vii) time-resolved fluorometric immunoassay (Malle, E., et al. (1995); J. Immunol. Methods 182, 131). See US Patent Number 6194163 which discloses a method for the quantitative measurement of human acute phase serum amyloid A protein.

The following experiments demonstrate that one or more of the biomarkers described herein can be used as accurate biomarker to monitor the response to anti-IL-13 therapies such as anti-IL-13 antibody therapy.

### EXAMPLE 1

### Microarray Experiment Description:

Peripheral blood mononuclear cells (PBMCs) were isolated from 8 healthy donors. These were cultured in RPMI + 10% FBS in the presence of IL-13 (10 ng/ml) or IL-4 (10 ng/ml) or CNTO 607 (10 µg/ml) or human IgG1 isotype control (10 µg/ml) for 24 hrs. Total RNA was isolated from the cells using the RNeasy kit (Qiagen). The quality and quantity of RNA was assessed using the bioanalyzer (Agilent) and microarray analysis was performed using the Affymatrix platform.

### Number of samples: 40

Chip type: Affymetrix GeneChip Human Genome U133 Plus 2.0 arrays were used in this study. Each array is comprised of more than 54K probe sets that analyze the expression level of over 47K transcripts and variants, including 38.5K well-characterized human genes.

### Number of chips: 40

### Data Processing and analysis:

Raw intensity data was downloaded from DNA Chip III database. Values below 0.01 were set to 0.01. Using GeneSpring (Redwood City, CA; version 7.2), chip-to-chip normalization was performed by dividing the averaged intensity of each probe set by the median intensity of a chip. The intensity of each probe set was then normalized to the median intensity of that probe set in the control group. The control groups in this study were the 8 untreated samples.

A probe set was regarded as reliably detected if it was called Present or Marginally present at least once among the 40 samples. Among 54675 probe sets on a chip, only 36357 probe sets passed the filtering and were analyzed further. Replicate samples were grouped according to their experimental conditions. The average of normalized intensities was used to represent each condition.

Using log 2 transformed normalized intensities, standard ANOVA was conducted in Partek Pro 6.1 (St. Charles, MO) to test treatment (untreated, IL13, IL4, CNTO 607, and isotype), and donor was also considered in the model as a random effect. Post-hoc tests were set up to identify genes showing significant differential expression between each treatment condition and untreated. False discovery rate cutoff was set at 0.05, meaning that 5% of identified genes would be false positives. Genes identified by statistical analysis were then filtered by fold change comparison between each treatment and untreated. The fold change cutoff was set at 1.5.

### Microarray Resullts:

From the above analysis of the microarray data the following list of genes was identified as potential biomarkers for IL-13. These include:
- Thymus and activation regulated chemokine (TARC) also known as CCL17
- Eotaxin 3 (CCL 26)
- Myeloid progenitor inhibitory factor 1 (MPIF-1), also known as CCL23
- Macrophage derived chemokine (MDC), also known as CCL22
- CD1A, CD1B, CD1C antigen
- WNT5A (wingless-type MMTV integration site family, member 5A)
- IgE CD23A
- hIL-17R beta

All of these genes were up regulated with IL-4 and IL-13 when compared to untreated but CNTO 607 or isotype control, as shown in Table 1, did not modulate their expression. For some of the genes more than one probe set was detected by microarray further confirming the results.

**Table 1: The raw and normalized intensities of each of the genes from microarray analysis and their Gen Bank accession numbers**

| **Gene (Probe set ID)** | **Treatment** | **Average Raw Intensity** | **Average Normalized Intensity** | **GenBank Accession** **No.** |
|---|---|---|---|---|
| **CCL17 (207900_at)** | Untreated | 138.8 | 1.086 | NM_002987 |
| | IL-13 | 2,268 | 17.62 | |
| | IL-4 | 2,870 | 22.42 | |
| | CNTO 607 | 130.8 | 1.005 | |
| | Isotype control | 177.1 | 1.41 | |
| **CCL26 (223710_at)** | Untreated | 41 | 1.64 | AF096296 |
| | IL-13 | 1883 | 72.26 | |
| | IL-4 | 1888 | 75.18 | |
| | CNTO 607 | 41.02 | 1.51 | |
| | Isotype control | 42.11 | 1.69 | |
| **CCL23 (210549_s_at)** | Untreated | 35.96 | 1.76 | U58913 |
| | IL-13 | 538.8 | 25.8 | |
| | IL-4 | 674.8 | 33.8 | |
| | CNTO 607 | 41.05 | 1.9 | |
| **Gene (Probe set ID)** | **Treatment** | **Average Raw Intensity** | **Average Normalized Intensity** | **GenBank Accession No.** |
| | Isotype control | 120.1 | 6.2 | |
| **CCL23 (210548_at)** | Untreated | 17.8 | 1.4 | U58913 |
| | IL-13 | 268.1 | 21.2 | |
| | IL-4 | 395.7 | 32.6 | |
| | CNTO 607 | 25 | 2.0 | |
| | Isotype control | 65.1 | 5.4 | |
| **CCL22 (207861_at)** | Untreated | 1550 | 1.2 | NM_002990 |
| | IL-13 | 4507 | 3.4 | |
| | IL-4 | 4326 | 3.3 | |
| | CNTO 607 | 1432 | 1 | |
| | Isotype control | 2636 | 2 | |
| **CD1A** | Untreated | 102.6 | 1 | M28825 |
| **(210325_at)** | | | | |
| | IL-13 | 433.3 | 4.3 | |
| | IL-4 | 586.6 | 6 | |
| | CNTO 607 | 116.4 | 1.1 | |
| | | | | |
| **Gene (Probe set ID)** | **Treatment** | **Average Raw Intensity** | **Average Normalized Intensity** | **GenBank Accession No.** |
| | Isotype control | 93.4 | 0.9 | |
| **CD1B (206749_at)** | Untreated | 104.7 | 1 | NM_001764 |
| | IL-13 | 1715 | 17.6 | |
| | IL-4 | 2142 | 22.5 | |
| | CNTO 607 | 92.9 | 0.9 | |
| | Isotype control | 84.62 | 0.9 | |
| **CD1C (205987_at)** | Untreated | 347.1 | 1 | NM_001765 |
| | IL-13 | 4251 | 12.79 | |
| | IL-4 | 4864 | 15.01 | |
| | CNTO 607 | 353 | 1 | |
| | Isotype control | 289.8 | 0.9 | |
| **WNT5A (205990_s_at)** | Untreated | 33.7 | 0.8 | NM_003392 |
| | IL-13 | 958.1 | 23.6 | |
| | IL-4 | 1091 | 27.1 | |
| | CNTO 607 | 40 | 0.9 | |
| | | | | |
| **Gene (Probe set ID)** | **Treatment** | **Average Raw Intensity** | **Average Normalized Intensity** | **GenBank Accession No.** |
| | Isotype control | 111.5 | 2.7 | |
| **WNT5A (213425_at)** | Untreated | 32.1 | 1.4 | NM_003392 |
| | IL-13 | 390.6 | 17.6 | |
| | IL-4 | 463.2 | 21 | |
| | CNTO 607 | 31.6 | 1.4 | |
| | Isotype control | 64.2 | 2.8 | |
| **WNT5A (231227_at)** | Untreated | 35.8 | 1.7 | NM_003392 |
| | IL-13 | 248.8 | 11.7 | |
| | IL-4 | 256.4 | 12.2 | |
| | CNTO 607 | 20.9 | 1 | |
| | Isotype control | 38 | 1.8 | |
| **IgE CD23A (206759_at)** | Untreated | 106.8 | 1 | NM_002002 |
| | IL-13 | 2811 | 28.4 | |
| | IL-4 | 3399 | 35.3 | |
| | CNTO 607 | 99.5 | 1 | |
| **Gene (Probe set ID)** | **Treatment** | **Average Raw Intensity** | **Average Normalized Intensity** | **GenBank Accession No.** |
| | Isotype control | 210.4 | 2.2 | |
| **IgE CD23A (206760_s_at)** | Untreated | 58.4 | 1.4 | NM_002002 |
| | IL-13 | 2091 | 49.4 | |
| | IL-4 | 2321 | 56.2 | |
| | CNTO 607 | 27.2 | 0.6 | |
| | Isotype control | 52.7 | 1.2 | |
| **hIL-17 R beta (219255_x_at)** | Untreated | 83.2 | 2.3 | NM_018725 |
| | IL-13 | 379.7 | 10.3 | |
| | IL-4 | 372.5 | 10.3 | |
| | CNTO 607 | 122.7 | 3.3 | |
| | Isotype control | 149.3 | 4.2 | |
| **hIL-17 R beta (224156_x_at)** | Untreated | 123.9 | 1.1 | NM_018725 |
| | IL-13 | 447 | 4 | |
| | IL-4 | 405.6 | 3.7 | |
| | CNTO 607 | 153.2 | 1.3 | |
| | | | | |
| **Gene (Probe set ID)** | **Treatment** | **Average Raw Intensity** | **Average Normalized Intensity** | **GenBank Accession No.** |
| | Isotype control | 180.2 | 1.6 | |
| **hIL-17 R beta (224361_s_at)** | Untreated | 100.9 | 1.2 | NM_018725 |
| | IL-13 | 362.9 | 4.5 | |
| | IL-4 | 340.2 | 4.3 | |
| | CNTO 607 | 128.3 | 1.6 | |
| | Isotype control | 145.5 | 1.8 | |

### Real Time PCR (TaqMan) confirmation:

In order to confirm the microarray finding by an independent means, Real Time PCR technology was employed. Total RNA from PBMCs cultured in the presence of IL-13 (10 ng/ml) or IL-4 (10 ng/ml) or CNTO 607 (10 µg/ml) or human IgG1 isotype control (10 µg/ml) for 24 hrs was reverse transcribed and used in Real Time PCR analysis for each of the 10 genes using the Applied Biosystems Gene Expression Assays on Demand on the ABI PRISM ® 7900HT Sequence Detection System. The results are summarized in Table 2.

**Table 2: The mean RNA quantity for each of the genes at various treatment conditions using TaqMan**

| **Gene** | **Treatment** | **Mean RNA quantity (Normalized to GAPDH)** | **Assay ID #** |
|---|---|---|---|
| **CCL17** | Untreated | 0.96 | Hs00171074_ml |
| | IL-13 | **289.6** | |
| | IL-4 | **489** | |
| | CNTO 607 | 0.96 | |
| | Isotype control | 2.4 | |
| **CCL26** | Untreated | Undetected | Hs00171146_ml |
| | IL-13 | **1.1** | |
| | IL-4 | **1.3** | |
| | CNTO 607 | Undetected | |
| | Isotype control | Undetected | |
| **CCL23** | Untreated | 0.57 | Hs00270756_ml |
| | IL-13 | **5.6** | |
| | IL-4 | **6.3** | |
| | CNTO 607 | 0.5 | |
| | Isotype control | 1.24 | |
| **CCL22** | Untreated | 1.4 | Hs00171080_ml |
| | IL-13 | **5.1** | |
| | IL-4 | **5.1** | |
| **Gene** | **Treatment** | **Mean RNA quantity (Normalized to GAPDH)** | **Assay ID #** |
| | CNTO 607 | 1.3 | |
| | Isotype control | 2.4 | |
| **CD1A** | Untreated | 1.1 | Hs00233332_ml |
| | IL-13 | **32.4** | |
| | IL-4 | **42.5** | |
| | CNTO 607 | 0.79 | |
| | Isotype control | 1 | |
| **CD1B** | Untreated | 1.3 | Hs00233507_ml |
| | IL-13 | **30.5** | |
| | IL-4 | **38.4** | |
| | CNTO 607 | 1.1 | |
| | Isotype control | 1.4 | |
| **CD1C** | Untreated | 0.9 | Hs00233509_ml |
| | IL-13 | **18.3** | |
| | IL-4 | **19.8** | |
| | CNTO 607 | 0.77 | |
| | Isotype control | 0.5 | |
| **WNT5A** | Untreated | 1.2 | Hs00180103_ml |
| | IL-13 | **147** | |
| | IL-4 | **190.4** | |
| **Gene** | **Treatment** | **Mean RNA quantity (Normalized to** GAPDH) | **Assay ID #** |
| | CNTO 607 | 2.1 | |
| | Isotype control | 7.2 | |
| **IgE CD23A** | Untreated | 0.79 | Hs00233627_ml |
| | IL-13 | **33.3** | |
| | IL-4 | **35.8** | |
| | CNTO 607 | 0.7 | |
| | Isotype control | 1.4 | |
| **hIL-17 R beta** | Untreated | 0.7 | Hs00218889_ml |
| | IL-13 | **6** | |
| | IL-4 | **5.8** | |
| | CNTO 607 | 0.7 | |
| | Isotype control | 1.1 | |

The Real Time PCR data seems to confirm the microarray data for each of the 10 genes.

### Protein analysis via ELISA:

To further confirm these data at the protein level a follow up study in PBMCs from 5 healthy donors was performed. PBMCs were cultured with IL-13 (10 ng/ml), IL-13 (10 ng/ml) plus anti-IL-13 therapeutic antibody, CNTO 607 (2 µg/ml) or IL-13 (10 ng/ml) plus isotype control mAb, human IgG1 (2 µg/ml) for 24 and 48 hr time points. The PBMC culture supernatant was assayed for TARC protein using a human TARC ELISA assay (R&D Systems) and for MDC protein using a human MDC ELISA assay (R&D Systems). The data showed that the neutralization of IL-13 by CNTO 607 resulted in a down regulation of IL-13 induced TARC and MDC at the protein level (Table 3). These data suggest that TARC and MDC could serve as potential biomarkers in an anti-IL-13 clinical study.

**Table 3: Mean protein level in PBMC culture supernatants from n=5 donors following various treatment conditions**

| **Treatment** | **Time point** | **TARC (pg/ml)** | **MDC (pg/ml)** |
|---|---|---|---|
| Untreated | 24 hr | 0.6 | 70.6 |
| IL-13 (10ng/ml) | 24 hr | 89 | 519.4 |
| IL-13 (10 ng/ml)+ human IgG1 (2µg/ml) | 24 hr | 86.2 | 473.6 |
| IL-13 (10 ng/ml)+ CNTO 607 (2 µg/ml) | 24 hr | 0.8 | 93.2 |
| Untreated | 48 hr | 2 | 110 |
| IL-13 (10ng/ml) | 48 hr | 287 | 851.2 |
| IL-13 (10 ng/ml)+ human IgG1 (2µg/ml) | 48 hr | 361.6 | 1398.2 |
| IL-13 (10 ng/ml)+ CNTO 607 (2 µg/ml) | 48 hr | 2.4 | 240.2 |

The role of biomarkers is becoming increasingly important in the clinical development of therapeutics. Their role ranges from, stratifying the patient population in helping to identify responders versus non-responders, to determining the efficacy of the therapeutic. Biomarkers can be a valuable tool in making better decisions that will reduce the cost for drug development and enable therapies to reach the right patient population faster.

Here we describe the use of the Microarray technology platform to identify biomarkers that are associated with the biology of IL-13, which could prove useful in an anti-IL-13 clinical trial. Peripheral blood mononuclear cells (PBMCs) from 10 healthy donors were cultured in the presence of IL-13, IL-4, anti-IL-13 mAb or an isotype control mAb and RNA from the treated cells was subjected to microarray. This revealed a number of genes that showed increased expression in the IL-13 and IL-4 treatment groups e.g., CCL17 (TARC), CCL22 (MDC), CCL23 (MPIF-1) and CCL26 (Eotaxin 3). These results were confirmed by Real Time PCR. A follow up study in PBMCs from five healthy donors that were cultured with IL-13, IL-13 plus anti-IL-13 mAb or IL-13 plus isotype control mAb showed that the neutralization of IL-13 resulted in a down regulation of IL-13 induced TARC, MDC and eotaxin 3 at the protein level. These data suggest that TARC, MDC and eotaxin 3 could serve as a potential efficacy specific biomarkers in an anti-IL-13 clinical study.

### EXAMPLE 2:

### Background

Asthma is a complex, chronic disorder, with a genetic and an environmental component (ST 1999). It is characterized by reversible airway obstruction, airway hyper-responsiveness, airway inflammation and remodeling (1997). Asthma affects an estimated 15 million Americans and the morbidity and mortality associated with it is on the rise in industrialized countries. Recent reports using murine models of allergic asthma have shown that the Th2 type cytokine IL-13 may play a critical role in the pathogenesis of asthma, either by regulating airway inflammation, mucus hyper-secretion or airway hyper-responsiveness (Grunig G 1998; Wills-Karp M 1998; Walter DM 2001; Venkayya R 2002; Akbari O 2003) thus making it an attractive target for therapeutic intervention.

The role of biomarkers is becoming increasingly important in the clinical development of therapeutics. A biomarker can be an indicator of normal biological processes, disease processes, or pharmacological responses to therapeutic intervention (2001).Their role ranges from, stratifying the patient population in helping to identify responders versus non-responders, to determining the efficacy of the therapeutic. Biomarkers can be a valuable tool in making better decisions that will reduce the cost for drug development and enable therapies to reach the right patient population faster.

The asthmatic population is very heterogeneous in terms of symptoms and response to therapy. Biomarkers can prove very useful in this by identifying responders versus non-responders. Thus leading to the development of more effective therapeutics. One approach uses the DNA Microarray platform to identify biomarkers using precilinical models. Here we describe the use of the Microarray technology platform to identify biomarkers that are associated with the biology of IL-13, which could prove useful in an anti-IL-13 clinical trial.

### Methods

### Peripheral blood mononuclear cell isolation and culture

Peripheral blood mononuclear cells (PBMCs) from healthy donors were isolated using Ficoll and density gradient centrifugation. The PBMCs were cultured in RPMI + 10% FBS and left untreated or treated with IL-13 (10 ng/ml) or IL-4 (10 ng/ml) or anti-IL-13 mAb (10 µg/ml) or isotype control mAb (10 µg/ml) for 24 hr.

### RNA isolation and microarray

Total cellular RNA was isolated from the cells using the RNeasy mini kit (Qiagen, Inc. Valencia, CA) as per manufacturer's instructions. The IL-13 and IL-4 was purchased from R&D Systems (Minneapolis, MN). The quality and quantity of RNA was assessed using the Agilent 2100 Bioanalyzer (South Plainfield, New Jersey). Samples that demonstrated high quality (ratio of 28S rRNA and 18S rRNA is greater than 1.7) were submitted for microarray analysis on the Affymatrix chip.

### Microarray Processing

RNA amplification, probe synthesis and labeling, cDNA chip hybridization and washing were performed as described previously [17]. Agilent Image Scanner was used to scan the cDNA chips (Agilent Technologies, Palo Alto, CA). Fluorescence intensity for each feature of the array was obtained by using ImaGene software (BioDiscovery, Los Angeles, CA).

### Microarray data analysis

In this study, Affymetrix GeneChip Human Genome U133 Plus 2.0 arrays were used to profile gene expression in human peripheral blood mononuclear cells from 10 donors stimulated with IL-13, or IL-4 at 1 time point (24 hr). Untreated samples from the same group of donors were used as control. Each array is comprised of more than 54K probe sets that analyze the expression level of over 47K transcripts and variants, including 38.5K well-characterized human genes. Raw intensity data was downloaded from DNA Chip III database. Values below 0.01 were set to 0.01. Using GeneSpring (Redwood City, CA; version 7.2), chip-to-chip normalization was performed by dividing the averaged intensity of each probe set by the median intensity of a chip. The intensity of each probe set was then normalized to the median intensity of that probe set in the control group. The control groups in this study were the 8 untreated samples.

A probe set was regarded as reliably detected if it was called Present or Marginally present at least once among the 40 samples. Among 54675 probe sets on a chip, only 36357 probe sets passed the filtering and were analyzed further. Replicate samples were grouped according to their experimental conditions. The average of normalized intensities was used to represent each condition.

Using log 2 transformed normalized intensities, standard ANOVA was conducted in Partek Pro 6.1 (St. Charles, MO) to test treatment (untreated, IL13, IL4, anti-IL-13 mAb, and isotype control mAb), and donor was also considered in the model as a random effect. Post-hoc tests were set up to identify genes showing significant differential expression between each treatment condition and untreated. False discovery rate cutoff was set at 0.05, meaning that 5% of identified genes would be false positives. Genes identified by statistical analysis were then filtered by fold change comparison between each treatment and untreated. The fold change cutoff was set at 1.5.

### Reverse Transcription and Real time PCR

1 µg of total RNA from each of the treated and untreated groups of PBMCs were used for the reverse transcription (RT) reaction. The RT reaction was performed as per protocol using TaqMan® RT reagents (Applied Biosystems) at 37 °C for 120 min followed by 25 °C for 10 min. Forty ng of cDNA per reaction were used in the Real Time PCR using the ABI Prism® 7900 sequence detection system (Foster City, California). In the presence of AmpliTaq Gold DNA plolymerase (ABI biosystem, Foster City, California), the reaction was incubated for 2 min at 50°C followed by 10 min at 95°C. Then the reaction was run for 40 cycles at 15 sec, 95°C and 1 min, 60°C per cycle. Assays-on-Demand™ primers and probes (Applied Biosystems) were used in the PCR. The Real Time PCR data was analyzed using the standard curve method.

### PBMC culture and ELISA

PBMCs were cultured in the presence of IL-13 (10 ng/ml) or IL-13 (10 ng/ml) + anti-IL-13 mAb (10 µg/ml) or IL-13 (10 ng/ml) + isotype control mAb (10 µg/ml). Culture media supernatants were collected and tested for levels of TARC, MDC and eotaxin 3 protein at 24 hr and 48 hr time points using human TARC specific, MDC specific and human eotaxin 3 specific ELISA as per the manufacturer's instructions (R&D Systems) Results

### IL-13 upregulates genes in PBMCs

In order to identify genes downstream of IL-13, which could serve as potential efficacy specific biomarkers cDNA microarray technology platform was used. IL-4 was also included in the treatment groups to try and differentiate between IL-13 and IL-4 and identify any markers that could be uniquely regulated by IL-13 alone.

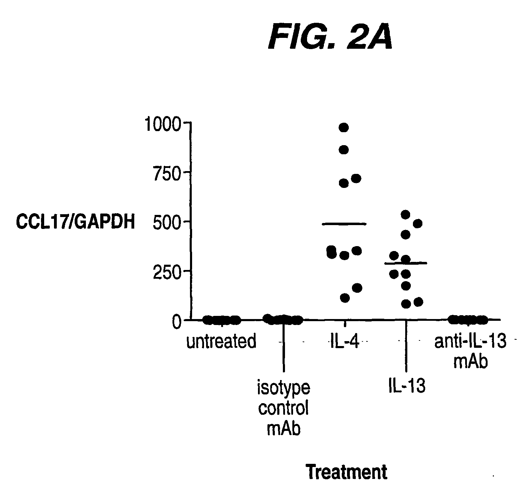

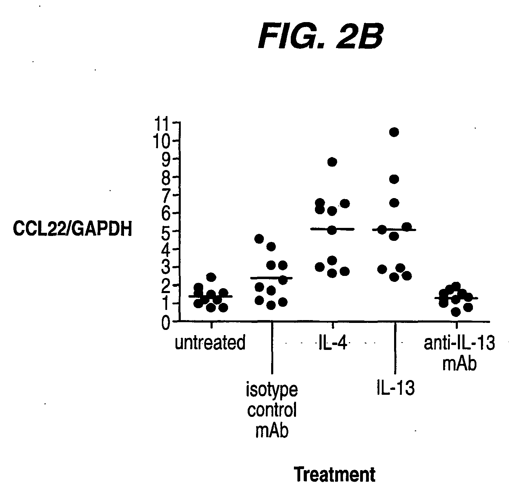

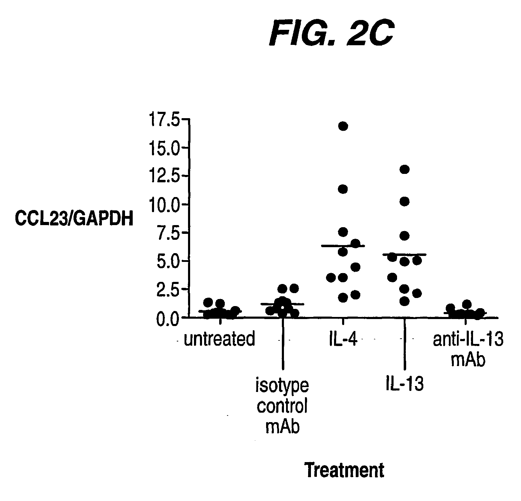

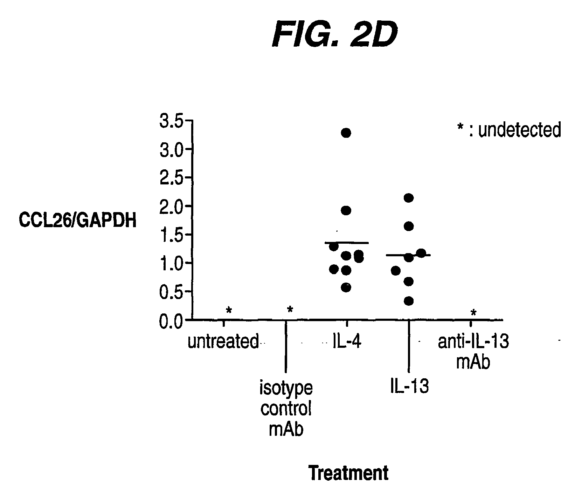

Ten donors were used and after analyzing the microarray data it became apparent that both IL-13 and IL-4 generated a very similar expression profile i.e., genes upregulated by IL-13 were the same as those upregulated by IL-4 at the 1.5 fold cutoff. From this a list of potential biomarkers was identified (Table 4). Figure 2 shows a graphical representation of the microarray data for four of them.

### Real Time PCR validation

TaqMan™ Real Time PCR was used to validate the potential biomrkers. As shown in Figure 2, CCL17, CCL22, CCL23 and CCL26 were all upregulated by IL-13 or IL-4 while their expression was not modulated by anti-IL-13 mAb or the isotype control mAb. This is very comparable to the microarray data.

### Validation of expression at protein level and inhibition of IL-13 induced protein by an anti-IL-13 mAb

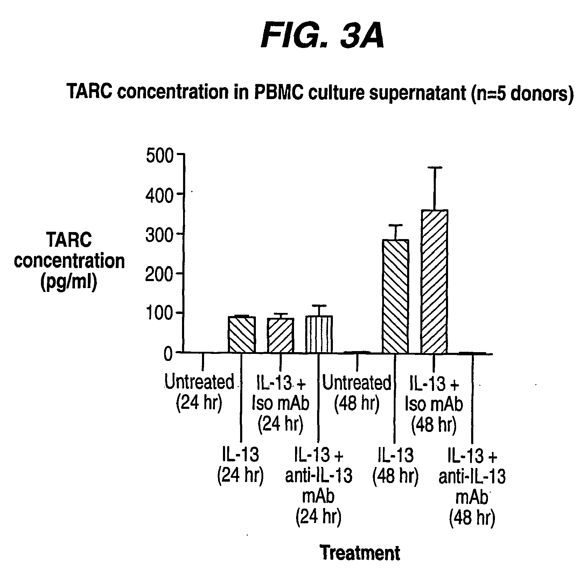

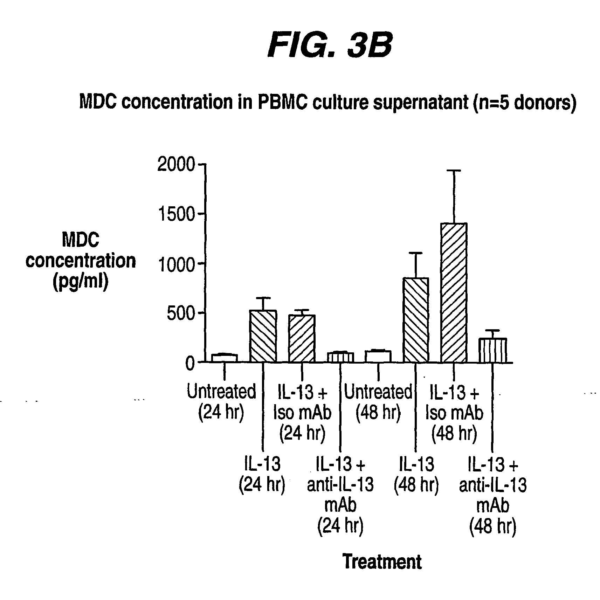

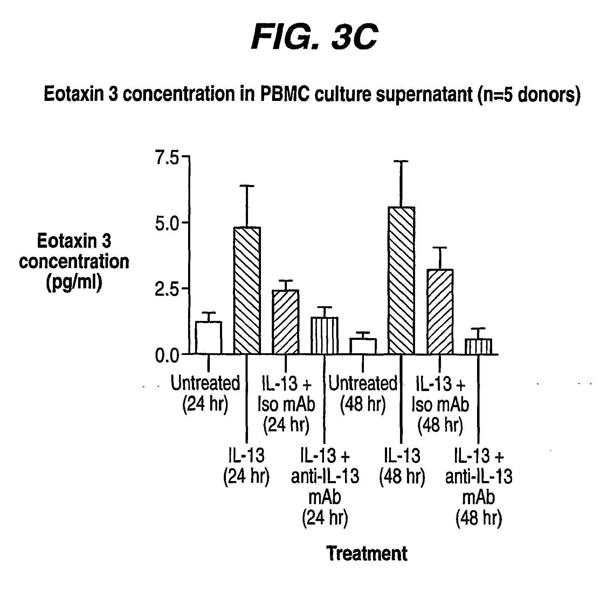

In order to validate the modulation of genes by IL-13 at the protein level, ELISA was used to detect potein in cell culture supernatants and confirm upregulation of CCL17, CCL22 and CCL26 by IL-13. As shown in Figure 3, IL-13 (10 ng/ml, 24 hr and 48 hr) stimulates the protein expression of CCL17, CCL22 and CCL26. Also neutralization of IL-13 by an anti-IL-13 mAb results in a down regulation of IL-13 induced CCL17, CCL22 and CCL26.

### Discussion

It is becoming increasing evident that there is a great need for biomarkers to become an integral part of drug discovery. Biomarkers can be especially valuable in helping to make early decisions on lead compounds and thereby drive down the cost of drug development. The microarray technology platform provides a unique approach in profiling the entire genome and thereby provide insight into potential markers that can help stratify a patient population or predict respone to therapy.

The asthmatic patient population is highly heterogenous both in terms of sysmptoms and response to therapy. Biomarkers can play a key role in identifying responders versus non-responders and thereby lead to the development of more effective therapies. A number of studies have been described where microarray platform has been used to identify asthma signature genes.

Here we describe the use of microarray technology to identify genes associated with the biology of IL-13.We have identified a panel of potential biomarkers, associated with IL-13 biology, which are down regulated upon neutralizing IL-13 and thus can be useful in an anti-IL-13 clinical trial. Such a panel of biomarkers can be used as a tool to monitor the efficacy of an anti-IL-13 therapeutic antibody.

The relevance of these genes to disease is very important for them to be effective biomarkers. To that end a number of the genes discussed above have been linked to diseases where IL-13 is believed to play a role. Increased level of TARC has been reported in serum and induced sputum of asthmatics (T Sekiya 2002), plasma of children during asthma exacerbation (Leung, Wong et al. 2003) and serum of patients with atopic dermatitis (Hijnen, de Bruin-Weller et al. 2004). This provides evidence that TARC might be involved in the pathogenesis of such disorders. Eotaxin 3 gene expression has been shown to be upregulated in bronchial biopsies of asthmatics after allergen challenge implying that eotaxin 3 may be important in late-phase eosinophil recruitment to the airways of asthmatics (Berkman, Ohnona et al. 2001). Also, CCL22 was shown to be up regulated in bronchoalveolar lavage (BAL) after a segmental allergen challenge of asthmatics(Pilette, Francis et al. 2004). Of the panel of potential biomarkers described above, WNT5A and MPIF-1 (CCL23) have not been previously reported to be regulated by IL-13. These are novel IL-13 related biomarkers, which can be useful in any disease where IL-13 plays a major role.

It is worth pointing out that he ultimate validity of a potential biomarker or panel of biomarkers can only be tested in the clinic. However, the purpose of this study and studies like this is to identify potential candidates which can be put forth for validation in a clinical setting.

**Table 4.**

| **Potential biomarkers associated with IL-13 biology** |
|---|
| CCL17 (TARC thymus & activation regulated chemokine) |
| CCL26 (Eotaxin 3) |
| CCL23 (MPIF-1 myeloid progenitor inhibitory factor 1) |
| CCL22 (MDC macrophage derived chemokine) |
| CD1 A,B,C |
| WNT5A |
| IgE CD23A |
| hIL-17 R beta |

### Figure Legends:

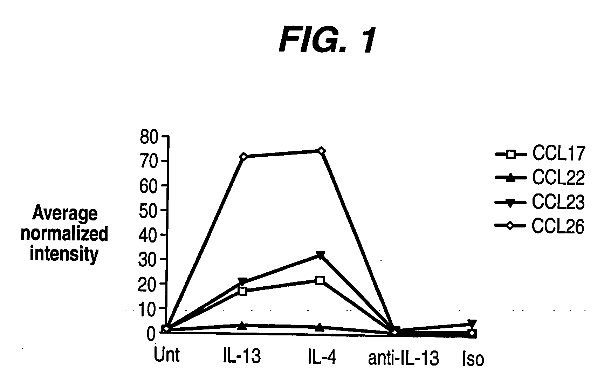

**Figure 1****.** Microarray data showing the average normalized intensity levels of CCL17, CCL22, CCL23 and CCL26 upon treatment of PBMCs from ten donors with IL-13, IL-4, anti-IL-13 mAb or isotype control mAb for 24 hrs.
**Figure 2****.** Real Time PCR (Taqman®) analysis showing the level of A) CCL17 B) CCL22 C) CCL23 D) CCL26 upon treatment of PBMCs from ten donors with IL-13, IL-4, anti-IL-13 mAb or isotype control mAb for 24 hrs. The quantity of each gene is normalized to GAPDH.
**Figure 3****.** Effect of neutralizing IL-13. PBMCs were incubated with IL-13, IL-13 + anti-IL-13 mAb, or IL-13 + isotype control mAb for 24 hr and 48 hr timepoints. The level of A) CCL17 B) MDC C) CCL26 was assessed in the cell culture supernatants using ELISA.

### References:

(1997). "National Institutes of Health. Guidelines for the diagnosis and management of asthma. Rep. No. 97-4051. Washington, DC: U.S. Dep. Health Human Serv., Natl. Heart Lung Blood Inst."
(2001). "Biomarkers and surrogate endpoints: preferred definitions and conceptual framework." Clin Pharmacol Ther 69(3): 89-95.
Akbari O, S. P., Meyer E, Kronenberg M, Sidobre S, Nakayama T, Taniguchi M, Grusby MJ, DeKruyff RH, Umetsu DT. (2003). "Essential role of NKT cells producing IL-4 and IL-13 in the development of allergen-induced airway hyperreactivity." Nat Med 9: 582-588.
Berkman, N., S. Ohnona, et al. (2001). "Eotaxin-3 but Not Eotaxin Gene Expression Is Upregulated in Asthmatics 24 Hours after Allergen Challenge." Am. J. Respir. Cell Mol. Biol. 24(6): 682-687.
Elias J, Z. A., Chupp G, Homer R. (1999). "Airway Remodeling in asthma." J Clin Invest 104(8): 1001-6.
Grunig G, W. M., Wakil AE, Venkayya R, Brombacher F, Rennick DM, Sheppard D, Mohrs M, Donaldson DD, Locksley RM, Corry DB. (1998). "Requirement for IL-13 independently of IL-4 in experimental asthma." Science 282: 2261-2263.
Hijnen, D., M. de Bruin-Weller, et al. (2004). "Serum thymus and activation-regulated chemokine (TARC) and cutaneous T cell-attracting chemokine (CTACK) levels in allergic diseases: TARC and CTACK are disease-specific markers for atopic dermatitis." Journal of Allergy and Clinical Immunology 113(2): 334-340.
Leung, T. F., C. K. Wong, et al. (2003). "Plasma TARC concentration may be a useful marker for asthmatic exacerbation in children." Eur Respir J 21(4): 616-620.
Pilette, C., J. N. Francis, et al. (2004). "CCR4 ligands are up-regulated in the airways of atopic asthmatics after segmental allergen challenge." Eur Respir J 23(6): 876-884.
Society, A. T. (2000). "Proceedings of the ATS workshop on refractory asthma: current understanding, recommendations, and unanswered questions." Am J Respir Crit Care Med 162(6): 2341-51.
ST, H. (1999). "The epidemic of allergy and asthma." Nature 402: B2-B4.
T Sekiya, H. Y., M Yamaguchi, K Yamamoto, A Ishii, O Yoshie, Y Sano, A Morita, K Matsushima, K Hirai (2002). "Increased levels of a TH2-type CC chemokine thymus and activation-regulated chemokine (TARC) in serum and induced sputum of asthmatics." Allergy 57(2): 173-177.
Venkayya R, L. M., Willkom M, Grunig G, Corry DB, Erle DJ. (2002). "The Th2 lymphocyte products IL-4 and IL-13 rapidly induce airway hyperresponsiveness through direct effects on resident airway cells." Am J Respir Cell Mol Biol 26: 202-208.
Walter DM, M. J., Berry G, McKenzie AN, Donaldson DD, DeKruyff RH, Umetsu DT. (2001). " Critical role for IL-13 in the development of allergen-induced airway hyperreactivity." J Immunol 167: 4668-4675.
Wills-Karp M, L. J., Xu X, Schofield B, Neben TY, Karp CL, Donaldson DD (1998). "Interleukin-13: central mediator of allergic asthma." Science 282: 2258-2261.

## Claims

1. A method of monitoring the response to anti-IL13 therapy in a patient undergoing such therapy by using one or more IL- 13 biomarkers, which comprises:
(i) Determining the level of expression of at least one of WNT5A or MPIF-1 (CCL23) in a tissue sample of a patient prior to administering the anti-IL-13 therapy to the patient;
(ii) Measuring the level of at least one of WNT5A or MPIF-1 (CCL23) expression in said tissue of the patient after having administered the anti-IL-13 therapy to the patient, and determining whether the anti-IL-13 therapy is effective in reducing the level of IL-13.

2. The method of claim 1 wherein the therapy is for the treatment of asthma, COPD or emphysema and said expression of at least one of WNT5A or MPIF-1 is employed as an IL-13 therapy responsive biomarker.

3. The method of any preceding claim wherein the anti-IL-13 therapy is selected from agents that neutralize or inhibit IL- 13.

4. The method of claim 3 wherein the anti-IL-13 therapy is an anti-IL-13 antibody or fusion protein.

5. The method of claim 4 wherein the anti-IL-13 therapy is the administration of an anti-IL-13 neutralizing antibody.

6. The method of any preceding claim, wherein said biomarkers optionally further include at least one of the biomarkers CCL17 (TARC), CCL22 (MDC), CCL23 (MPIF-1) and CCL26 (Eotaxin 3), CD1A, CD1B, CDIC, IgE CD23A, IL-17Rbeta.

## Patentansprüche

1. Verfahren zum Verfolgen des Ansprechens eines Patienten auf anti-IL-13-Therapie, bei dem eine solche Therapie durchgeführt wird, unter Verwendung von einem oder mehr IL-13-Biomarkern, das Folgendes umfasst:
(i) Bestimmen des Expressionsniveaus von mindestens einem von WNT5A oder MPIF-1(CCL23) in einer Gewebeprobe eines Patienten vor der Verabreichung der anti-IL-13-Therapie an den Patienten;
(ii) Bestimmen des Ausmaßes der Expression von mindestens einem von WNT5A oder MPIF-1 (CLL23) in dem Gewebe des Patienten nach der Verabreichung der anti-IL-13-Therapie an den Patienten, und Bestimmen, ob die anti-IL-13-Therapie eine Erniedrigung der IL-13-Menge bewirkt.

2. Verfahren nach Anspruch 1, wobei die Therapie der Behandlung von Asthma, COPD oder Emphysem dient und die Expression von mindestens einem von WNT5A oder MPIF-1 als Biomarker für das Ansprechen auf IL-13-Therapie verwendet wird.

3. Verfahren nach einem vorhergehenden Anspruch, wobei die anti-IL-13-Therapie aus der Reihe der Mittel, die IL-13 neutralisieren oder inhibieren, ausgewählt wird.

4. Verfahren nach Anspruch 3, wobei es sich bei der anti-IL-13-Therapie um einen anti-IL-13-Antikörper oder ein Fusionsprotein handelt.

5. Verfahren nach Anspruch 4, wobei es sich bei der anti-IL-13-Therapie um die Verabreichung eines anti-IL-13-neutralisierenden Antikörpers handelt.

6. Verfahren nach einem vorhergehenden Anspruch, wobei die Biomarker gegebenenfalls weiterhin mindestens einen der Biomarker CCL17 (TARC), CCL22 (MDC), CCL23 (MPIF-1) und CCL26 (Eotaxin 3), CDIA, CDIB, CDIC, IgE CD23A, IL-17Rbeta beinhalten.

## Revendications

1. Méthode de suivi de la réponse à la thérapie anti-IL-13 chez un patient recevant une telle thérapie en utilisant un ou plusieurs biomarqueurs IL-13, qui comprend :
(i) la détermination du taux d'expression d'au moins l'un parmi WNT5A ou MPIF-1(CCL23) dans un prélèvement de tissu d'un patient avant l'administration de la thérapie anti-IL-13 au patient ;
(ii) la mesure du taux d'expression d'au moins l'un parmi WNT5A ou MPIF-1 (CCL23) dans ledit tissu du patient après avoir administré la thérapie anti-IL-13 au patient, et le fait de déterminer si la thérapie anti-IL-13 est efficace pour réduire le taux d'IL-13.

2. Méthode selon la revendication 1, **caractérisée en ce que** la thérapie est pour le traitement de l'asthme, de la BPCO ou de l'emphysème et ladite expression d'au moins l'un parmi WNT5A ou MPIF-1 est utilisée en tant que biomarqueur de réponse à la thérapie IL-13.

3. Méthode selon l'une ou l'autre des revendications précédentes, **caractérisée en ce que** la thérapie anti-IL-13 est choisie parmi les agents qui neutralisent ou inhibent IL-13.

4. Méthode selon la revendication 3, **caractérisée en ce que** la thérapie anti-IL-13 est un anticorps anti-IL-13 ou une protéine de fusion.

5. Méthode selon la revendication 4, **caractérisée en ce que** la thérapie anti-IL-13 est l'administration d'un anticorps neutralisant anti-IL-13.

6. Méthode selon l'une quelconque des revendications précédentes, **caractérisée en ce que** lesdits biomarqueurs incluent éventuellement en outre au moins l'un des biomarqueurs CCL17 (TARC), CCL22 (MDC), CCL23 (MPIF-1) et CCL26 (Eotaxine 3), CDIA, CDIB, CDIC, IgE CD23A, IL-17Rbêta.
